(19) Europäisches Patentamt
European Patent Office
Office européen des brevets

(11) **EP 2 662 429 B1**

(12) **EUROPEAN PATENT SPECIFICATION**

(45) Date of publication and mention of the grant of the patent:
**10.06.2015 Bulletin 2015/24**

(21) Application number: **11854571.4**

(22) Date of filing: **12.08.2011**

(51) Int Cl.:
*C09J 133/02* *(2006.01)*       *A61K 8/02* *(2006.01)*
*A61K 9/06* *(2006.01)*       *C09J 7/00* *(2006.01)*
*C09J 11/04* *(2006.01)*       *C09J 11/06* *(2006.01)*
*C09J 139/06* *(2006.01)*

(86) International application number:
**PCT/JP2011/068451**

(87) International publication number:
**WO 2012/093505 (12.07.2012 Gazette 2012/28)**

(54) **COMPOSITION FOR ADHESIVE HYDROGEL AND USE THEREOF**

ZUSAMMENSETZUNG FÜR EIN HAFTFÄHIGES HYDROGEL UND VERWENDUNG DAVON

COMPOSITION POUR UN HYDROGEL ADHÉSIF ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.01.2011 JP 2011001407**

(43) Date of publication of application:
**13.11.2013 Bulletin 2013/46**

(73) Proprietor: **Sekisui Plastics Co., Ltd.**
**Osaka-shi, Osaka 530-0047 (JP)**

(72) Inventors:
• **SATO, Kaori**
**Tenri-shi**
**Nara 632-0007 (JP)**
• **HATORI, Takaaki**
**Tenri-shi**
**Nara 632-0007 (JP)**
• **FUJIWARA, Yasuhiro**
**Tenri-shi**
**Nara 632-0007 (JP)**

(74) Representative: **Hoffmann Eitle**
**Patent- und Rechtsanwälte PartmbB**
**Arabellastraße 30**
**81925 München (DE)**

(56) References cited:
**WO-A2-2004/093786       JP-A- 5 207 984**
**JP-A- 2002 501 804       JP-A- 2007 112 972**
**JP-A- 2009 227 924**

## Description

### TECHNICAL FIELD

The present invention relates to a composition for an adhesive hydrogel showing adhesiveness suitable for repeated use and being less irritating to the skin, in particular, and a use thereof (for example, adhesive hydrogel, gel sheet and electrode).

### BACKGROUND ART

Basically, a hydrogel is a highly hydrophilic polymer swelling in an aqueous medium. The hydrogel has various properties such as water absorbing properties, swelling tendency, adhesiveness and conductivity. Taking advantage of such properties, the hydrogel has been used in a wide range of fields including civil engineering and construction, agriculture, food, medical care, cosmetics and electricity.

In medical practice, for example, the adhesive hydrogel is used as electrode pads of a measuring apparatus such as an electrocardiogram. In recent years, the adhesive hydrogel has been used as electrode pads of an EMS (Electrical Muscle Stimulation) for the purpose of weight reduction and muscle training. The EMS is exercise equipment for contracting muscles using electrical stimulation by attaching electrode pads made of an adhesive hydrogel to the skin, and it is increasingly used also in ordinary households.

The adhesive hydrogel to be used for such electrode pads is required to have high adhesiveness to the skin.

For example, Patent Document 1 discloses a technique for improving the adhesiveness of a hydrogel by using a specified amount of water-soluble polymer and medical electrodes with the use of the hydrogel. Although the technique disclosed in Patent Document 1 can improve the adhesive strength of the hydrogel, the adhesive strength is soon reduced after attachment and detachment are repeated several times. That is, the adhesive strength is not sufficient for repeated use. In addition, the hydrogel causes the skin to turn reddish after being detached from the skin.

The adhesive strength is reduced after repeated use and the skin turns reddish mainly because cuticle is separated from a skin surface and adheres to a surface of an electrode pad when the electrode pad is detached from the skin, for example, and therefore the effective adhesive area is reduced.

As a method for maintaining the adhesive strength in repeated use, for example, Patent Document 2 discloses a technique of recovering the adhesive strength by rinsing with water an adhesive surface whose adhesive strength has been reduced due to attachment to and detachment from the skin repeated several times.

Since the hydrogel described in Patent Document 2 requires an action such as rinsing with water, however, users feel it complicated to have to comply with requirements set forth in an instruction manual as to how to wash and how to dry the hydrogel in order to certainly recover the adhesive strength.

Furthermore, the object of preventing the skin from turning reddish, that is, the object of soothing irritation to the skin has not been achieved at present.

### RELATED ART DOCUMENTS

### PATENT DOCUMENTS

[Patent Document 1] Japanese Unexamined Patent Publication No. 2003-96431
[Patent Document 2] Japanese Patent No. 3437124

### SUMMARY OF THE INVENTION

### PROBLEMS TO BE SOLVED BY THE INVENTION

In view of the above-described existing circumstances, it is an object of the present invention to provide: a composition for an adhesive hydrogel having excellent adhesiveness in repeated use without requiring a special action such as rinsing with water and being less irritating to the skin, in particular; and a use thereof.

### MEANS FOR SOLVING THE PROBLEMS

The present invention is directed to a composition for an adhesive hydrogel, comprising at least: a polymeric matrix former; water; and a polyhydric alcohol; the polymeric matrix former comprising at least: (a) (meth)acrylic acid, a (meth)acrylic acid derivative or a vinyl derivative having 2 carboxyl groups and 4 or 5 carbon atoms; (b) (meth)acrylamide or a (meth)acrylamide derivative; (c) N-vinyl-2-caprolactam and/or N-vinyl-2-valerolactam; and (d) a cross-linkable monomer, wherein the content percentage of the (b) in the composition for an adhesive hydrogel is 2 to 20% by weight.

Hereinafter, the present invention will be described in detail.

## EFFECTS OF THE INVENTION

An adhesive hydrogel made of a composition for an adhesive hydrogel of the present invention has high adhesiveness to the skin, has excellent adhesiveness in repeated use without requiring a special action such as rinsing with water and is less irritating to the skin, in particular. Accordingly, products such as electrode pads with the use of the adhesive hydrogel of the present invention are more durable against repeated use than conventional products and therefore economical as having an extended product lifetime, and contribute to resources-saving.

## MODE FOR CARRYING OUT THE INVENTION

The composition for an adhesive hydrogel of the present invention comprises at least a polymeric matrix former, water and a polyhydric alcohol.

The polymeric matrix former forms a polymeric matrix when the composition for an adhesive hydrogel of the present invention is reacted to prepare an adhesive hydrogel.

In the present invention, the polymeric matrix former comprises at least: (a) (meth)acrylic acid, a (meth)acrylic acid derivative or a vinyl derivative having 2 carboxyl groups and 4 or 5 carbon atoms; (b) (meth)acrylamide or a (meth)acrylamide derivative; (c) N-vinyl-2-caprolactam and/or N-vinyl-2-valerolactam; and (d) a cross-linkable monomer.

The above-mentioned (a) (meth)acrylic acid, a (meth)acrylic acid derivative or a vinyl derivative having 2 carboxyl groups and 4 or 5 carbon atoms has a role to form a skeleton of the adhesive hydrogel. Since these compounds have a high affinity for the other monomers to be described later as well as have good polymerization reactivity, it is possible to reduce residual monomers.

The above-mentioned (a) (meth)acrylic acid, a (meth)acrylic acid derivative or a vinyl derivative having 2 carboxyl groups and 4 or 5 carbon atoms is not particularly limited and examples thereof include (meth)acrylic acid, maleic acid, fumaric acid, itaconic acid, crotonic acid, (poly)ethyleneglycol (meth)acrylate, (poly)propyleneglycol (meth)acrylate, (poly)glycerin (meth)acrylate and the like. For a reason of solubility to the water and to the polyhydric alcohol, compounds having water-solubility are particularly preferable. These compounds may be used independently, or two or more kinds may be used in combination.

Here, the (meth)acrylic acid means acrylic acid or methacrylic acid, and the (poly)ethylene means ethylene or polyethylene. The polyethylene means a structure including 2 to 10 ethylene units. The (poly)propylene and the (poly)glycerin mean in the same manner as in the (poly)ethylene.

The content percentage of the above-mentioned (a) (meth)acrylic acid, a (meth)acrylic acid derivative or a vinyl derivative having 2 carboxyl groups and 4 or 5 carbon atoms in the composition for an adhesive hydrogel of the present invention is not particularly limited. A preferable lower limit of the content percentage is 2% by weight, and a preferable upper limit of the content percentage is 20% by weight. When the content percentage of (a) is less than 2% by weight, the adhesiveness of the adhesive hydrogel to be obtained will be insufficient, and the adhesive hydrogel may not be fixed to the skin, in particular. When the content percentage of (a) is more than 20% by weight, there will be an unreacted material, and therefore the step of a following process may be complicated or the quality of the adhesive hydrogel to be obtained may be reduced. A more preferable lower limit of the content percentage of (a) is 8% by weight, and a more preferable upper limit thereof is 15% by weight.

The above-mentioned (b) (meth)acrylamide or a (meth)acrylamide derivative has a role to give the adhesive hydrogel to be obtained adhesive strength for sufficient fixation to the skin, in particular, and to reduce skin troubles by preventing the stratum corneum from separating from the skin when the adhesive hydrogel is detached from the skin. It also has a role to maintain adhesiveness of the adhesive hydrogel in repeated use in order to prevent the separation of the stratum corneum.

The above-mentioned (b) (meth)acrylamide or a (meth)acrylamide derivative is not particularly limited, and examples thereof include non-electrolyte acrylamide derivatives such as tert-butylacrylamide sulfonic acid (TBAS), tert-butylacrylamide sulfonate, N,N-dimethyl aminoethyl acrylamide (DMAEAA) hydrochloride, N,N-dimethyl amino propyl acryl amide (DMAPAA) hydrochloride, (meth)acrylamide, N-methyl(meth)acrylamide, N-ethyl(meth)acrylamide, N-propyl(meth)acrylamide, N,N-dimethyl(meth)acrylamide, N,N-diethyl(meth)acrylamide, acryloyl morpholine and the like. In particular, for reasons of solubility to the water and to the polyhydric alcohol, and of readiness for polymerization reaction with other monomers, compounds having water-solubility are preferable. These compounds may be used independently, or two or more kinds may be used in combination.

The lower limit of the content percentage of the above-mentioned (b) (meth)acrylamide or a (meth)acrylamide derivative in the composition for an adhesive hydrogel of the present invention is 2% by weight, and the upper limit thereof is 20% by weight. When the content percentage of (b) is less than 2% by weight, the adhesiveness of the adhesive hydrogel to be obtained will be insufficient, and the adhesive hydrogel may not be fixed to the skin, in particular. In addition, despite its insufficient adhesiveness, the adhesive hydrogel causes skin troubles and undergoes significant reduction in adhesive strength when detached from the skin, although the reason therefor is not unexplained. Furthermore, there will be an unreacted material from the other components, and therefore the step of a following process will be complicated or the quality of the adhesive hydrogel to be obtained will be reduced. When the content percentage of the (b) is more than 20% by weight, the adhesive hydrogel causes skin troubles and undergoes significant reduction in adhesive strength when detached from the skin, because the detachment is accompanied by separation of the stratum corneum. A preferable lower limit of the content percentage of the (b) is 3% by weight, and a preferable upper limit thereof is 10% by weight.

The above-mentioned (c) N-vinyl-2-caprolactam and/or N-vinyl-2-valerolactam has a role to adjust the viscosity and the adhesive strength of the adhesive hydrogel to be obtained, to reduce skin troubles by preventing the stratum corneum from separating from the skin when the adhesive hydrogel is detached from the skin, and to maintain adhesiveness of the adhesive hydrogel in repeated use.

The content percentage of the above-mentioned (c) N-vinyl-2-caprolactam and/or N-vinyl-2-valerolactam in the composition for an adhesive hydrogel of the present invention is not particularly limited. A preferable lower limit thereof is 0.1% by weight, and a preferable upper limit thereof is 25% by weight. When the content percentage of the (c) is less than 0.1% by weight, the adhesive hydrogel may cause skin troubles and undergo significant reduction in adhesive strength when detached from the skin, because the detachment is accompanied by separation of the stratum corneum. When the content percentage of the (c) is more than 25% by weight, the adhesiveness of the adhesive hydrogel to be obtained will be insufficient, and the adhesive hydrogel may not be fixed to the skin, in particular. A more preferable lower limit of the content percentage of the (c) is 0.2% by weight, and a more preferable upper limit thereof is 20% by weight.

The above-mentioned (d) a cross-linkable monomer is not particularly limited, and a monomer having two or more double bonds having a polymerizable property in a molecule is preferable. Examples thereof include methylenebis(meth)acrylamide, ethylenebis(meth)acrylamide, (poly)ethyleneglycol di(meth)acrylate, (poly)propyleneglycol di(meth)acrylate, 1,9-nonanediol di(meth)acrylate, glycerin di(meth)acrylate, glycerin tri(meth)acrylate, tetraaryloxy ethane, diaryl ammonium chloride and the like. These compounds may be used independently, or two or more kinds may be used in combination.

In addition, as the cross-linkable monomer, glycidyl (meth)acrylate, N-methylol acrylamide and the like having one or more polymerizable double bonds and one or more functional groups having cross-linkable reactivity by an elimination reaction or a ring cleavage reaction such as epoxy group, hydroxyl group and alkoxy group are also suitably used.

The content percentage of the above-mentioned (d) a cross-linkable monomer in the composition for an adhesive hydrogel of the present invention is not particularly limited. A preferable lower limit thereof is 0.01% by weight, and a preferable upper limit thereof is 0.5% by weight. When the content percentage of the cross-linkable monomer is less than 0.01% by weight, the cross-linking density is reduced and therefore the form stability of the adhesive hydrogel is reduced. As a result, the adhesive hydrogel cannot be obtained or the adhesive hydrogel may be broken when detached from the skin. In addition, the thickness of the adhesive hydrogel varies to be likely to result in variation of measurement values when the adhesive hydrogel of the present invention is used for an electrical measurement. When the content percentage of the cross-linkable monomer is more than 0.5% by weight, the adhesiveness of the adhesive hydrogel to be obtained will be insufficient, and therefore fixation of the adhesive hydrogel to the skin may be difficult or the adhesive hydrogel may be fragile, having no plasticity. A more preferable lower limit of the content percentage of the cross-linkable monomer is 0.04% by weight, and a more preferable upper limit thereof is 0.1% by weight.

The composition for an adhesive hydrogel of the present invention may contain (e) polyvinylpyrrolidone or a polyvinylpyrrolidone derivative as a component of the polymeric matrix former. By including the (e), it is possible to improve the adhesiveness and elasticity of the adhesive hydrogel.

The content percentage of the (e) in the composition for an adhesive hydrogel of the present invention is not particularly limited. A preferable lower limit thereof is 0.1% by weight, and a preferable upper limit thereof is 20% by weight. When the content percentage of the (e) is less than 0.1% by weight, an effect by containing the (e) will not be sufficiently obtained. When the content percentage of the (e) is more than 20% by weight, the composition for an adhesive hydrogel will be inhomogeneous, and therefore the adhesive hydrogel may not be obtained, or even if obtained, the adhesiveness of the adhesive hydrogel may be nonuniform. A more preferable lower limit of the content percentage of the (e) is 0.2% by weight, and a more preferable upper limit thereof is 15% by weight.

The content percentage of the polymeric matrix former in the composition for an adhesive hydrogel of the present invention is not particularly limited. A preferable upper limit thereof is 50% by weight. When the content percentage of the polymeric matrix former is more than 50% by weight, too much heat will be generated during the polymerization, and therefore a runaway reaction may be caused and a solvent may boil by heating to a temperature exceeding its boiling point. In the case of the boiling, bubbles may be mixed in the adhesive hydrogel. A more preferable upper limit of the content percentage of the polymeric matrix former is 40% by weight. The preferable lower limit of the content percentage of the polymeric matrix former is not particularly limited and may be adjusted as appropriate according to the ranges of the content percentages of the above-mentioned components.

The composition for an adhesive hydrogel of the present invention contains a polyhydric alcohol.

The polyhydric alcohol is not particularly limited and examples thereof include ethyleneglycol, propylene glycol, butanediol, glycerin, pentaerythritol, sorbitol, polyethylene glycol, polypropylene glycol, polyglycerol, polyoxyethylene polyglyceryl ether and the like. These compounds may be used independently, or two or more kinds may be used in combination.

Especially, it is preferable to use a polyhydric alcohol which is in a liquid state in a temperature range in which the adhesive hydrogel is used (for example, around 20°C, when the adhesive hydrogel is used indoors). Specifically, ethyleneglycol, propylene glycol, glycerin, polyethylene glycol and polyglycerol are suitable.

The content percentage of the polyhydric alcohol in the composition for an adhesive hydrogel is not particularly limited. A preferable lower limit thereof is 10% by weight, and a preferable upper limit thereof is 80% by weight. When the content percentage of the polyhydric alcohol is less than 10% by weight, the moisture retaining ability of the adhesive hydrogel to be obtained will be poor to result in significant moisture loss. As a result, the adhesive hydrogel may lack temporal stability and flexibility, and therefore sufficient adhesiveness may not be obtained. When the content percentage of the polyhydric alcohol is more than 80% by weight, the polyhydric alcohol may bleed out from a surface of the adhesive hydrogel to be obtained, and the adhesive strength of the adhesive hydrogel may be reduced. In addition, bubbles will be easily mixed in during the preparation of the adhesive hydrogel. A more preferable lower limit of the content percentage of the polyhydric alcohol is 20% by weight, and a more preferable upper limit thereof is 70% by weight.

The composition for an adhesive hydrogel of the present invention contains water.

The content percentage of the water is not particularly limited. A preferable lower limit thereof is 10% by weight, and a preferable upper limit thereof is 50% by weight. When the content percentage of the water is less than 10% by weight, the moisture absorption of the adhesive hydrogel to be obtained will be increased, and the adhesive hydrogel may change in quality with time. When the content percentage of the water is more than 50% by weight, the adhesive hydrogel may contract or change in physical properties due to desiccation. A more preferable lower limit of the content percentage of the water is 12% by weight, and a more preferable upper limit thereof is 30% by weight.

Preferably, the composition for an adhesive hydrogel of the present invention contains an electrolyte. By including an electrolyte, it is possible to obtain a conductive adhesive hydrogel suitable for the use as a bioelectrode such as electrodes for measuring electrocardiogram, electrodes for low-frequency therapy equipment and various ground electrodes. Preferably, the adhesive hydrogel has a specific resistance of 0.1 to 10 kΩ.cm when used as such a bioelectrode.

In addition, the electrolyte may be used as a pH adjuster, an enhancer of the moisture retaining ability of the adhesive hydrogel or an antimicrobial agent.

The electrolyte is not particularly limited, and examples thereof include alkali metal halides, alkali earth metal halides and other metal halides such as sodium halide, potassium halide, magnesium halide and calcium halide;

salts of inorganic acids such as hypochlorous acid, chlorous acid, chloric acid, perchloric acid, sulfuric acid, nitric acid and phosphoric acid with various metals or ammonium salts of these inorganic acids;

various complex salts;

salts of monovalent organic carboxylic acids such as acetic acid, benzoic acid and lactic acid with various metals or ammonium salts of these monovalent organic carboxylic acids;

salts of polyvalent carboxylic acids such as phthalic acid, succinic acid, adipic acid, citric acid and tartaric acid with one or more metals or with ammonia;

salts of organic acids such as sulfonic acid and amino acid with various metals or ammonium salts of these organic acids; and

salts of polyacids such as poly(meth)acrylic acid, polyvinylsulfonic acid, poly tert-butylacrylamido sulfonic acid, polyallylamine and polyethyleneimine with various metals or ammonium salts of these polyacids (polyelectrolyte salts).

When the electrolyte is included in the composition for an adhesive hydrogel of the present invention for the purpose of imparting appropriate conductivity to the adhesive hydrogel, a preferable lower limit of the content percentage of the electrolyte in the composition for an adhesive hydrogel of the present invention is 0.05% by weight, and a preferable upper limit thereof is 10% by weight. The hydrogel containing water originally has properties as a dielectric. When such a hydrogel is combined with an electrode element to give an electrode, the hydrogel will have an electric capacity according to the gel thickness and to the electrode element area. However, the impedance (Z) of the electrode is largely influenced by the electrolytic concentration particularly in a low frequency range lower than 1 kHz. When the content percentage of the electrolyte is less than 0.05% by weight, the impedance will be so high that the hydrogel may not be suitable for the use as a conductive material. When the content percentage of the electrolyte is more than 10% by weight, it will be difficult for the electrolyte to dissolve in the hydrogel, and therefore crystals may be deposited inside the gel or other components may be prevented from dissolving. Besides, the conductivity will reach the limit, and therefore further addition of the electrolyte is not beneficial in terms of conductivity imparting. A more preferable lower limit of the content percentage of the electrolyte is 0.1% by weight, and a more preferable upper limit thereof is 2% by weight.

Preferably, the composition for an adhesive hydrogel of the present invention contains a polymerization initiator. The polymerization initiator is not particularly limited, and examples thereof include a photo radical polymerization initiator and a thermal radical polymerization initiator.

The photo radical polymerization initiator is not particularly limited, and examples thereof include $\alpha$-hydroxyketone, $\alpha$-aminoketone, benzylmethyl ketal, bisacylphosphineoxide, metallocene and the like. More specifically, may be mentioned 1-[4-(2-hydroxyethoxy)-phenyl]-2-hydroxy-2-methyl-propane-1-one (product name: Irgacure 2959, produced by Ciba Specialty Chemicals K.K.), 2-hydroxy-2-methyl-1-phenyl-propane-1-one (product name: Darocur 1173, produced by Ciba Specialty Chemicals K.K.), 1-hydroxy-cyclohexyl-phenyl-ketone (product name: Irgacure 184, produced by Ciba Specialty Chemicals K.K.), 2-methyl-1-[(methylthio)phenyl]-2-morpholinopropane-1-one (product name: Irgacure 907, produced by Ciba Specialty Chemicals K.K.), 2-benzyl-2-dimethylamino-1-(4-morpholinophenyl)-butane-1-one (product name: Irgacure 369, produced by Ciba Specialty Chemicals K.K.) and the like.

These photo radical polymerization initiators may be used independently, or two or more kinds may be used in combination.

The thermal radical polymerization initiator is not particularly limited, and examples thereof include organic peroxides such as benzoyl peroxide; azo-based polymerization initiators such as azobisisobutyronitrile; persulfates such as potassium persulfate and ammonium persulfate; azo compounds such as 2,2-azobisamidinopropane dihydrochloride and the like. In addition, a redox initiator such as hydrogen peroxide and sodium thiosulfate may be used in combination with the polymerization initiator as needed.

The content percentage of the polymerization initiator in the composition for an adhesive hydrogel of the present invention is not particularly limited. A preferable lower limit thereof is 0.01% by weight, and a preferable upper limit thereof is 1% by weight. When the content percentage of the polymerization initiator is less than 0.01% by weight, the polymerization reaction may not progress sufficiently. When the content percentage of the polymerization initiator is more than 1% by weight, the adhesive hydrogel to be obtained may be discolored (yellow discoloration) or given odor by polymerization initiator residue after the polymerization reaction. A more preferable lower limit of the content percentage of the polymerization initiator is 0.05% by weight, and a more preferable upper limit thereof is 0.5% by weight.

To the composition for an adhesive hydrogel of the present invention, a base such as sodium hydroxide may be added as appropriate for the purpose of controlling pH.

The composition for an adhesive hydrogel of the present invention may further contain additives such as an antiseptic agent, a disinfectant, a rust preventive agent, an antioxidant, a stabilizer, a fragrant material, a surfactant, a coloring agent, an anti-inflammatory medication, a vitamin preparation and a whitening agent as needed.

The method for preparing an adhesive hydrogel using the composition for an adhesive hydrogel of the present invention is not particularly limited, and examples thereof include a method by performing heating or ultraviolet light irradiation on the composition for an adhesive hydrogel of the present invention.

An adhesive hydrogel prepared by using the composition for an adhesive hydrogel of the present invention, comprising at least a polymer matrix obtained by polymerization of the polymeric matrix former, water and a polyhydric alcohol is also an aspect of the present invention.

The adhesive hydrogel of the present invention can also be obtained by impregnating the polymer matrix formed beforehand by the polymerization reaction with water or a non-cross-linked water-soluble polymer. Alternatively, the adhesive hydrogel of the present invention can be obtained by a method comprising: adding a cross-linkable monomer or a catalyst to a polymer formulation obtained by dissolving or homogeneously dispersing a linear polymer obtained by polymerizing only polymerizable monomers, a non-cross-linked water-soluble polymer and so on in water; and reacting the linear polymer and the cross-linkable monomer to give a polymer matrix.

When the adhesive hydrogel of the present invention is prepared by ultraviolet light irradiation, the cumulative ultraviolet light dosage is preferably 1000 mJ/cm$^2$ to 10000 mJ/cm$^2$, and more preferably 2000 mJ/cm$^2$ to 10000 mJ/cm$^2$.

Preferably, the adhesive hydrogel of the present invention has an adhesive strength of 0.9 N or higher, where the adhesive hydrogel is cut out into a strip shape of 20 mm $\times$ 100 mm and lined with a nonwoven fabric to give a test piece, and the test piece is attached to a bakelite plate (bakelite sheet, product number: PL113, produced by Sumitomo Bakelite Co., Ltd.) and set in Tensilon (produced by Orientec Co., Ltd.), and strength needed to detach the test piece at an angle of 90° at a speed of 300 mm/minute according to JIS-Z0237 is defined as the adhesive strength. Having an adhesive strength of 0.9 N or higher, the adhesive hydrogel of the present invention has a sufficient adhesive strength even when used as a bioelectrode, in particular.

Preferably, the adhesive hydrogel of the present invention has adhesive strengths N1 and N10, which are determined by the following method, of 0.5 N or higher. When both the N1 and the N10 are 0.5 N or higher, the adhesive hydrogel of the present invention can be sufficiently fixed to the skin, in particular, and therefore considered as having excellent adhesive strength for repeated use.

The N 1 and the N10 are determined as follows. That is, the adhesive hydrogel is cut out into a strip shape of 20 mm × 100 mm and lined with a nonwoven fabric ("spun laced nonwoven #8021", produced by Du Pont Kabushiki Kaisha) to give a test piece. This test piece is attached to the skin of the medial side of the left arm softly wiped with alcohol in advance. After the attachment for two minutes, strength needed to detach the test piece at an angle of 180° at a speed of 1000 mm/minute is measured with Tensilon (produced by Orientec Co., Ltd.) This value is defined as an initial adhesive strength N1. Next, the test piece detached is attached to the skin of the medial side of the left arm, and then detached. This process is repeated. Strength needed to detach the test piece for the 10th time counting from the first detachment is measured. This value is determined as N10.

When a numerical value obtained by dividing the N10 by the N1 (N10/N1) is defined as an adhesive strength retention relative to the initial adhesive strength, the adhesive strength retention (N10/N1) is preferably 0.8 or higher.

Reduction of the adhesive strength is attributed to the stratum corneum having separated from the skin and adhering to the surface of the adhesive hydrogel. When the adhesive strength retention (N10/N1) is 0.8 or higher, the adhesive hydrogel of the present invention reduces separation of the stratum corneum from the skin, being less irritating to the skin.

Here, in the present description, the measurement of the N1 and the N10 was performed on the medial sides of the left arms of six subjects (one male and one female in their 20's, one male and one female in their 40's, and one male and one female in their 50's) under an atmosphere with a temperature of 23°C and a relative humidity of 60%, and the measurement results were averaged.

(Exemplification of method for forming adhesive hydrogel (gel sheet))

Since a formulation in a liquid state is used for the polymerization and the cross-linking to cause gelation of the adhesive hydrogel, the adhesive hydrogel can be formed as appropriate according to its use. For example, the composition is applied dropwise onto a base film such as a resin film, and subsequently a top film such as a resin film is overlaid on an upper surface of the composition (dropwise) to force the composition to spread and to be controlled to have a desired thickness. In this state, the polymerization and the cross-linking are caused by light (ultraviolet light) irradiation and/or heating, and thereby an adhesive hydrogel having a desired thickness can be obtained.

When the hydrogel is used as an adhesive, for example, it may be formed into a gel sheet having a thickness of 0.01 mm to 2.0 mm. In this case, it may be formed into a gel sheet provided with a protective film on one side or on both sides.

The protective film for forming the gel sheet can be used as a separator or a support, and commonly known protective films may be used. The method for forming the gel sheet is not particularly limited; the gel sheet may be formed by any commonly known methods.

As the base film, polyester, polyolefin, polystyrene, polyurethane, paper or paper laminated with a resin film, and the like can be used. Preferably, a surface of the base film which is in contact with the gel sheet is subjected to a release agent treatment such as silicone coating.

When the base film is used as a release paper, the base film obtained by release-agent treating the surface of a film such as polyester, polyolefin, polystyrene, paper or paper laminated with a resin film is suitably used. Biaxially stretched PET film, OPP and the like are particularly preferable. Examples of the release agent treatment include silicone coating. In particular, silicone coating of a baking type, in which a coating material is cross-linked and cured by heat or ultraviolet light, is preferable.

When the base film is used as a packing material (lining material) of the adhesive rather than as the release paper, it is preferable to use polyester, polyolefin, polystyrene, polyurethane or the like without the release agent treatment. Especially, polyurethane films are particularly preferable since they have flexibility and some of them have water vapor permeability. Since the polyurethane films alone are usually too flexible and difficult to handle in production steps, they are preferably laminated with polyolefin, paper and the like as a carrier film for use.

Preferably, the step of generating gel is performed while the base film is being provided with the carrier film. Basically, the same materials as for the base film can be used for the top film. However, it is preferable to select a film of a material for the top film that does not block light in order not to prevent photopolymerization. It is not preferable that a film which is used as the packing material (lining material) is used as the top film. It is not preferable particularly when the film which is used as the packing material is likely to be deteriorated by ultraviolet light irradiation or the like, because the film will be placed on a side to be directly irradiated with ultraviolet light.

(Exemplification of electrode pad with the use of hydrogel and method for preparing the same)

The adhesive hydrogel has properties of safely adhering to the skin and the like. In addition, the adhesive hydrogel can be easily given conductivity by optionally adding an electrolyte thereto. Accordingly, the adhesive hydrogel can be suitably used as an adhesive conductive part of an electrode pad or the like. Hereinafter, an example of the use of the adhesive hydrogel of the present invention as an electrode pad will be described.

First, the adhesive hydrogel is stacked on one side of a conductive layer incorporating a conductive material such as carbon. Then, a nonconductive support is stacked on the other side of the conductive layer having the hydrogel stacked thereon. A conductive material such as a copper wire is attached to the support so as to be in contact with the conductive layer and serve as a terminal. Electricity is applied from an external or to an external monitor or the like through the terminal. Thus, an adhesive conductive part can be formed which is capable of applying electricity to a subject through the conductive layer and the hydrogel.

As the conductive layer, a carbon coating layer formed of a carbon paste including polyester type or polyurethane type resin as a binder, a layer formed by printed coating of conductive ink containing a metal such as Ag/AgCl, carbon or the like, a layer formed by laminating a resin film with a metal foil (aluminum, stainless steel, Ag or the like) and with a conductive film incorporating carbon or the like, and the like may be used. Preferably, the conductive layer maintains enough strength so that it will not be easily cut when a force such as pulling force is exerted thereon from without. If the conductive layer is easily cut, the conductive layer may be cut during the preparation of an electrode, or the conductive layer may be elongated to result in deformation of an electrode as an end product, or the conductive layer may be broken in an electrode and induce burn injury depending on the user's handling. On the other hand, the conductive layer needs to have flexibility, because it is to be attached to the skin surface, which is rough. It is necessary to select a conductive layer having physical properties that prevent troubles in use.

In the case of a film formed of a laminate of the support and the conductive layer, the terminal is formed by leaving a portion of the film not overlaid with the adhesive hydrogel when stacking the adhesive hydrogel on the conductive layer. The terminal (portion not overlaid with the adhesive hydrogel) is clipped and connected to a lead wire.

In the case of an electrode having a calking structure, the terminal is not particularly limited as long as it has conductivity. In this case, a portion having a structure held between an element formed by coating a resin molded article with a metal such as Ag/AgCl and a snap terminal made of a resin molded article incorporating a metal such as stainless steel and carbon serves as the terminal to be connected to the external.

In the case where a metal wire is used, the metal wire has a structure in which a portion thereof is held between the conductive layer and the support, and a portion thereof outside the laminate of the conductive layer and the support is covered with nonconductive resin or the like, and an end of the metal wire is connected to a lead wire thereby to be connected to an external.

Since the adhesive hydrogel of the present invention has excellent adhesive strength in repeat use as described above, a gel sheet or an electrode pad obtained by using the hydrogel has excellent adhesive strength in repeated use unlike any conventional products, and therefore the product life thereof can be extended. It is therefore expected that the frequency of disposal is reduced, being contributory to resources-saving. In addition, the adhesive hydrogel of the present invention is less likely to redden the skin when detached from the skin. That is, it is less irritating to the skin.

EXAMPLES

Hereinafter, the present invention will be described in detail with reference to examples. However, the present invention is not limited to the examples.

(Example 1)

As polymerizable monomers, 14.4 parts by weight of acrylic acid, 9.6 parts by weight of tert-butylacrylamide sulfonic acid (TBAS) and 3 parts by weight of N-vinyl-2-caprolactam; as a cross-linkable monomer, 0.04 parts by weight of N,N'-methylene bisacrylamide (MBAA); as a polyhydric alcohol, 44 parts by weight of glycerin; 3 parts by weight of polyvinylpyrrolidone (CREEJUS K30, produced by Dai-ichi Kogyo Seiyaku Co., Ltd., K-value: 29.2); as an electrolyte, 0.5 parts by weight of sodium chloride; and as an ultraviolet polymerization initiator, 0.2 parts by weight of 1-[4-(2-hydroxyethoxy)-phenyl]-2-hydroxy-2-methyl-propane-1-one (product name: Irgacure 2959, produced by Ciba Specialty Chemicals K.K.) were stirred and dissolved to give a monomer mixed solution.

Thereafter, the mixed solution was adjusted to have a pH of 4.0 to 5.0 with 8 parts by weight of 50% by weight NaOH aqueous solution, and ion-exchange water was added thereto until the amount of water was 17.3 parts by weight. Next, the resulting monomer mixed solution was irradiated with ultraviolet light having an energy amount of 3000 mJ/cm$^2$ with a metal halide lamp to give a sheet-like adhesive hydrogel having a thickness of 0.75 mm.

(Examples 2 to 16 and Comparative Examples 1 to 9)

Adhesive hydrogels were prepared in the same manner as in Example 1 except that the composition was varied according to the compositions shown in Tables 1 to 3. It was impossible to shape, in Comparative Example 6 and Comparative Example 9.

In Tables 1 to 3, DMAA refers to N,N-dimethylacrylamide, and 1,9ND refers to 1,9-nonanediol dimethacrylate.

(Method for measuring adhesive strength)

Each adhesive hydrogel prepared was cut out into a strip shape of 20 mm × 100 mm and lined with a nonwoven fabric ("spun laced nonwoven #8021", produced by Du Pont Kabushiki Kaisha, thickness: 0.38 mm) to give a test piece, and the test piece was attached to a bakelite plate (bakelite sheet, product number: PL113, produced by Sumitomo Bakelite Co., Ltd.) and set in Tensilon (produced by Orientec Co., Ltd.) Then, the test piece was detached at an angle of 90° at a speed of 300 mm/minute according to JIS-Z0237, and the strength needed therefor was determined as the adhesive strength. Tables 1 to 3 show the results.

(Method for measuring adhesive strength in repeated use: 180° adhesive strength in repeated use to skin)

Each adhesive hydrogel prepared was cut out into a strip shape of 20 mm × 100 mm and lined with a nonwoven fabric ("spun laced nonwoven #8021", produced by Du Pont Kabushiki Kaisha, thickness: 0.38 mm) to give a test piece. The test piece was attached to the skin of the medial side of a left arm softly wiped with alcohol in advance. After the attachment for two minutes, strength needed to detach the test piece at an angle of 180° at a speed of 1000 mm/minute was measured with Tensilon (produced by Orientec Co., Ltd.). This value was determined as the initial adhesive strength N 1. Next, the test piece detached was attached to the skin of the medial side of the left arm, and then detached. This process was repeated. Strength needed to detach the test piece for the 10th time counting from the first detachment was measured. This value was determined as the N10. A numerical value obtained by dividing the N10 by the N1 (N10/N1) was determined as an adhesive strength retention relative to the initial adhesive strength.
The measurement of the N1 and the N10 was performed on the medial sides of the left arms of six subjects (one male and one female in their 20's, one male and one female in their 40's, and one male and one female in their 50's) under an atmosphere with a temperature of 23°C and a relative humidity of 60%, and the measurement results were averaged.
The N10 of Comparative Example 1 was too low to measure.
Furthermore, after the test piece was detached for the 10th time, occurrence of redness on the skin surface was evaluated by visual observation. When the redness occurred in at least one of the six subjects, the test piece was evaluated as "observed".

(Method for measuring specific resistance)

Each adhesive hydrogel prepared was cut out into a square shape of 20 mm × 20 mm and lined with a nonwoven fabric to give a test piece, and stainless plates were attached to both sides of the test piece. An impedance when a current was applied between the stainless plates at 1 kHz, 10 Vp-p was measured. The specific resistance was calculated by substituting the impedance (I) obtained, and an area (S) and a thickness (d) of the test piece into the following equation.

$$\text{Specific resistance } (\Omega.cm) = I \ (\Omega) \ / \ d \ (cm) \times S \ (cm^2)$$

(Elasticity test on adhesive hydrogel)

Each adhesive hydrogel obtained was cut out into a piece of 50 mm × 50mm, and a central region of a surface of the piece was pressed with a thumb for five seconds to form a recess in the gel surface, and then the thumb was released. A time required for the surface to return to its original state was measured. The evaluation was made according to the following criteria.
Good : Time to return to original state was shorter than five seconds.
Acceptable : Time to return to original state was five seconds or longer and shorter than 30 seconds.
Poor : Time to return to original state was 30 seconds or longer.

[Table 1]

| | Components | Composition | Examples | | | | | | | |
|---|---|---|---|---|---|---|---|---|---|---|
| | | | 1 | 2 | 3 | 4 | 5 | 6 | 7 | 8 |
| Blending ratio [wt%] | a | Acrylic acid | 14.4 | 14.4 | 12.3 | 12.3 | 9.2 | 9.2 | 14.4 | 14.4 |
| | b | TBAS | 9.6 | 9.6 | 3.1 | 3.1 | 6.1 | 6.1 | 9.6 | 9.6 |
| | | DMAA | 0 | 0 | 0 | 0 | 0 | 0 | 0 | 0 |
| | c | N-vinyl-2-caprolactam | 3.0 | 3.0 | 1.9 | 1.9 | 1.9 | 1.9 | 0.5 | 3.0 |
| | d | MBAA | 0.04 | 0.06 | 0.06 | 0.07 | 0.06 | 0.08 | 0.04 | 0.04 |
| | | 1,9ND | 0 | 0 | 0 | 0 | 0 | 0 | 0 | 0 |
| | e | Polyvinylpyrrolidone | 3.0 | 3.0 | 1.9 | 1.9 | 1.9 | 1.9 | 3.0 | 0.5 |
| | Polyhydric alcohol | Glycerin | 44 | 44 | 51 | 51 | 51 | 51 | 44 | 44 |
| | Water | | 17.3 | 17.2 | 23.0 | 23.0 | 23.1 | 23.1 | 19.8 | 19.8 |
| | Initiator | | 0.2 | 0.2 | 0.2 | 0.2 | 0.2 | 0.2 | 0.2 | 0.2 |
| | Electrolyte | NaCl | 0.5 | 0.5 | 0.5 | 0.5 | 0.5 | 0.5 | 0.5 | 0.5 |
| | 50% NaOH | | 8.0 | 8.0 | 6.0 | 6.0 | 6.0 | 6.0 | 8.0 | 8.0 |
| Evaluation | Thickness | | 0.75 | 0.75 | 0.75 | 0.75 | 0.75 | 0.75 | 0.75 | 0.75 |
| | Adhesive strength [N] | | 4.79 | 4.56 | 2.20 | 1.45 | 1.39 | 1.24 | 1.25 | 1.19 |
| | Adhesive strength in repeated use | N1 | 1.38 | 1.35 | 1.27 | 1.24 | 1.37 | 1.14 | 1.11 | 1.01 |
| | | N10 | 1.28 | 1.23 | 1.14 | 1.10 | 1.23 | 1.00 | 0.96 | 0.88 |
| | | N10/N1 | 0.93 | 0.91 | 0.90 | 0.89 | 0.90 | 0.88 | 0.87 | 0.87 |
| | Skin redness after N10 | | Not observed | Not observed | Not observed | Not observed | Not observed | Not observed | Not observed | Not observed |
| | Specific resistance [kΩ.cm] | | 1.6 | 1.2 | 1.2 | 1.6 | 1.1 | 1.3 | 1.4 | 1.3 |
| | Elasticity | | Good | Good | Good | Good | Good | Good | Good | Good |

EP 2 662 429 B1

Table 2

| | Components | Composition | Examples | | | | | | | |
|---|---|---|---|---|---|---|---|---|---|---|
| | | | 9 | 10 | 11 | 12 | 13 | 14 | 15 | 16 |
| Blending ratio [wt %] | a | Acrylic acid | 8.1 | 11.5 | 14.4 | 14.4 | 14.4 | 14.4 | 14.4 | 25 |
| | b | TBAS | 6.9 | 7.6 | 9.6 | 9.6 | 9.6 | 0 | 9.6 | 16 |
| | | DMAA | 0 | 0 | 0 | 0 | 0 | 9.6 | 0 | 0 |
| | c | N-vinyl-2-caprolactam | 12 | 20 | 3.0 | 3.0 | 3.0 | 3.0 | 3.0 | 3.0 |
| | d | MBAA | 0.04 | 0.06 | 0.04 | 0.04 | 0.06 | 0.04 | 0 | 0.04 |
| | | 1,9ND | 0 | 0 | 0 | 0 | 0 | 0 | 0.04 | 0 |
| | e | Polyvinylpyrrolidone | 12 | 0 | 3.0 | 3.0 | 0 | 3.0 | 3.0 | 3.0 |
| | Polyhydric alcohol | Glycerin | 40 | 35 | 44 | 44 | 44 | 44 | 44 | 34 |
| | Water | | 14.3 | 18.6 | 17.3 | 17.3 | 20.2 | 17.3 | 17.3 | 10.7 |
| | Initiator | | 0.2 | 0.2 | 0.2 | 0.20 | 0.2 | 0.2 | 0.2 | 0.2 |
| | Electrolyte NaCl | | 0.5 | 0.5 | 0.5 | 0.5 | 0.5 | 0.5 | 0.5 | 0.5 |
| | 50% NaOH | | 6.0 | 6.5 | 8.0 | 8.0 | 8.0 | 8.0 | 8.0 | 8.0 |
| Evaluation | Thickness | | 0.75 | 0.75 | 0.75 | 0.75 | 0.75 | 0.75 | 0.75 | 0.75 |
| | Adhesive strength [N] | | 3.09 | 3.20 | 1.00 | 0.97 | 3.20 | 0.92 | 1.09 | 0.90 |
| | Adhesive strength in repeated use | N1 | 1.03 | 1.13 | 0.81 | 0.76 | 0.95 | 0.75 | 1.01 | 0.76 |
| | | N10 | 1.18 | 0.98 | 0.71 | 0.66 | 0.86 | 0.65 | 0.88 | 0.61 |
| | | N10/N1 | 0.91 | 0.87 | 0.86 | 0.86 | 0.90 | 0.86 | 0.87 | 0.80 |
| | Skin redness after N10 | | Not observed | Not observed | Not observed | Not observed | Not observed | Not observed | Not observed | Not observed |
| | Specific resistance (kΩ.cm) | | 1.2 | 1.4 | 1.5 | 1.4 | 1.2 | 2.1 | 1.6 | 1.2 |
| | Elasticity | | Good | Acceptable | Good | Good | Acceptable | Good | Good | Acceptable |

EP 2 662 429 B1

[Table 3]

| Components | | Composition | Comparative Examples | | | | | | | | |
|---|---|---|---|---|---|---|---|---|---|---|---|
| | | | 1 | 2 | 3 | 4 | 5 | 6 | 7 | 8 | 9 |
| Blending ratio [wt %] | a | Acrylic acid | 14.4 | 14.4 | 14.4 | 9.3 | 1.5 | 0 | 14.4 | 14.4 | 14.4 |
| | b | TBAS | 0 | 1.0 | 25 | 1.0 | 0.5 | 10.6 | 9.6 | 9.6 | 9.6 |
| | | DMAA | 0 | 0 | 0 | 0 | 0 | 0 | 0 | 0 | 0 |
| | c | N-vinyl-2-caprolactam | 3.0 | 3.0 | 3.0 | 3.0 | 25 | 3.0 | 0 | 0 | 3.0 |
| | d | MBAA | 0.04 | 0.04 | 0.04 | 0.04 | 0.04 | 0.04 | 0.06 | 0.06 | 0 |
| | | 1,9ND | 0 | 0 | 0 | 0 | 0 | 0 | 0 | 0 | 0 |
| | e | Polyvinylpyrrolidone | 0 | 3.0 | 3.0 | 3.0 | 3.0 | 3.0 | 3.0 | 0 | 0 |
| | Polyhydric alcohol | Glycerin | 44 | 40 | 40 | 40 | 44 | 40 | 44 | 44 | 40 |
| | Water | | 29.9 | 29.9 | 5.9 | 35.0 | 24.6 | 34.7 | 20.2 | 23.2 | 24.3 |
| | Initiator | | 0.2 | 0.2 | 0.2 | 0.2 | 0.2 | 0.2 | 0.2 | 0.2 | 0.2 |
| | Electrolyte | NaCl | 0.5 | 0.5 | 0.5 | 0.5 | 0.5 | 0.5 | 0.5 | 0.5 | 0.5 |
| | 50% NaOH | | 8.0 | 8.0 | 8.0 | 8.0 | 0.7 | 8.0 | 8.0 | 8.0 | 8.0 |
| Evaluation | Thickness | | 0.75 | 0.75 | 0.75 | 0.75 | 0.75 | Not shapable | 0.75 | 0.75 | Not shapable |
| | Adhesive strength [N] | | 0.40 | 0.68 | 3.60 | 0.45 | 0.88 | - | 1.09 | 0.97 | - |
| | Adhesive strength in repeated use | N1 | 0.30 | 0.46 | 1.80 | 0.31 | 0.55 | - | 0.95 | 0.77 | - |
| | | N10 | Not measurable | 0.12 | 0.90 | 0.27 | 0.45 | - | 0.61 | 0.54 | - |
| | | N10/N1 | - | 0.25 | 0.50 | 0.86 | 0.82 | - | 0.64 | 0.70 | - |
| | Skin redness after N10 | | Not observed | Observed | Observed | Not observed | Not observed | - | Observed | Observed | - |
| | Specific resistance [kΩ.cm] | | 1.3 | 1.3 | 1.6 | 1.2 | 1.1 | - | 1.5 | 1.3 | - |
| | Elasticity | | Acceptable | Acceptable | Acceptable | Acceptable | Poor | - | Good | Acceptable | - |

EP 2 662 429 B1

It has been confirmed that the adhesive hydrogels prepared in Examples 1 to 16 have superior adhesiveness in repeated use without requiring a special action such as rinsing with water and are less irritating to the skin, in particular. It has been also confirmed that the adhesive hydrogels can be improved in elasticity and will be less likely to be damaged even by repeated use, when the component (e) is contained (Examples 1 to 9, 11, 12, 14 and 15).

In Example 16, an adhesive hydrogel acceptable in terms of properties was obtained, but a slight amount of residue was observed after the polymerization. The residue was no longer observed after the polymerization was performed again (after the ultraviolet light irradiation was performed again). This was assumedly because the amount of the component (a) was too much.

**Claims**

1. A composition for an adhesive hydrogel, comprising at least: a polymeric matrix former; water; and a polyhydric alcohol; the polymeric matrix former comprising at least:

    (a) (meth)acrylic acid, a (meth)acrylic acid derivative or a vinyl derivative having 2 carboxyl groups and 4 or 5 carbon atoms;
    (b) (meth)acrylamide or a (meth)acrylamide derivative;
    (c) N-vinyl-2-caprolactam and/or N-vinyl-2-valerolactam; and
    (d) a cross-linkable monomer,
    wherein the content percentage of the (b) in the composition for an adhesive hydrogel is 2 to 20% by weight.

2. The composition for an adhesive hydrogel of claim 1, wherein the content percentage of the (a) is 2 to 20% by weight, and the content percentage of the (c) is 0.1 to 25% by weight.

3. The composition for an adhesive hydrogel of claim 1, wherein the polymeric matrix former contains the (e) polyvinylpyrrolidone or a polyvinylpyrrolidone derivative.

4. The composition for an adhesive hydrogel of claim 3, wherein the content percentage of the (e) is 0.1 to 20% by weight.

5. The composition for an adhesive hydrogel of claim 1, wherein the polyhydric alcohol is at least one kind selected from group consisting of ethylene glycol, propylene glycol, butanediol, glycerin, pentaerythritol, sorbitol, polyethylene glycol, polypropylene glycol, polyglycerol and polyoxyethylene polyglyceryl ether.

6. The composition for an adhesive hydrogel of claim 1, wherein the (a) is at least one kind selected from group consisting of (meth)acrylic acid, maleic acid, fumaric acid, itaconic acid, crotonic acid, (poly)ethylene glycol (meth)acrylate, (poly)propylene glycol (meth)acrylate and (poly)glycerin (meth)acrylate.

7. The composition for an adhesive hydrogel of claim 1, wherein the (b) is at least one kind selected from group consisting of tert-butylacrylamide sulfonic acid (TBAS), tert-butylacrylamide sulfonate, N,N-dimethyl aminoethyl acrylamide (DMAEAA) hydrochloride, N,N-dimethyl amino propyl acryl amide (DMAPAA) hydrochloride, (meth)acrylamide, N-methyl(meth)acrylamide, N-ethyl(meth)acrylamide, N-propyl(meth)acrylamide, N,N-dimethyl(meth)acrylamide, N,N-diethyl(meth)acrylamide and acryloyl morpholine.

8. The composition for an adhesive hydrogel of claim 1, wherein the (d) is at least one kind selected from group consisting of methylenebis(meth)acrylamide, ethylenebis(meth)acrylamide, (poly)ethyleneglycol di(meth)acrylate, (poly)propyleneglycol di(meth)acrylate, 1,9-nonanediol di(meth)acrylate, glycerin di(meth)acrylate, glycerin tri(meth)acrylate, tetraaryloxy ethane and diaryl ammonium chloride.

9. The composition for an adhesive hydrogel of claim 1 further comprising an electrolyte.

10. An adhesive hydrogel prepared by using the composition for the adhesive hydrogel of claim 1, comprising at least a polymer matrix obtained by polymerization of the polymeric matrix former, water and a polyhydric alcohol.

11. A gel sheet prepared by using the adhesive hydrogel of claim 10.

12. An electrode prepared by using the adhesive hydrogel of claim 10.

**Patentansprüche**

1. Zusammensetzung für ein haftendes Hydrogel, die zumindest einen polymeren Matrixbildner, Wasser und einen mehrwertigen Alkohol umfasst, wobei der polymere Matrixbildner zumindest umfasst:

    (a) (Meth)acrylsäure, ein (Meth)acrylsäurederivat oder ein Vinylderivat mit 2 Carboxylgruppen und 4 oder 5 Kohlenstoffatomen;
    (b) (Meth)acrylamid oder ein (Meth)acrylamidderivat;
    (c) N-Vinyl-2-caprolactam und/oder N-Vinyl-2-valerolactam; und
    (d) ein vernetzbares Monomer,
    wobei der prozentuale Gehalt an (b) in der Zusammensetzung für ein haftendes Hydrogel 2 bis 20 Gew.% beträgt.

2. Zusammensetzung für ein haftendes Hydrogel gemäss Anspruch 1, wobei der prozentuale Gehalt an (a) 2 bis 20 Gew.% beträgt und der prozentuale Gehalt an (c) 0,1 bis 25 Gew.% beträgt.

3. Zusammensetzung für ein haftendes Hydrogel gemäss Anspruch 1, wobei der polymere Matrixbildner (e) Polyvinylpyrrolidon oder ein Polyvinylpyrrolidonderivat enthält.

4. Zusammensetzung für ein haftendes Hydrogel gemäss Anspruch 3, wobei der prozentuale Gehalt an (e) 0,1 bis 20 Gew.% beträgt.

5. Zusammensetzung für ein haftendes Hydrogel gemäss Anspruch 1, wobei der mehrwertige Alkohol zumindest eine Art, ausgewählt aus der Gruppe bestehend aus Ethylenglykol, Propylenglykol, Butandiol, Glycerin, Pentaerythrit, Sorbit, Polyethylenglykol, Polypropylenglykol, Polyglycerin und Polyoxyethylenpolyglycerylether, ist.

6. Zusammensetzung für ein haftendes Hydrogel gemäss Anspruch 1, wobei (a) zumindest eine Art, ausgewählt aus der Gruppe bestehend aus (Meth)acrylsäure, Maleinsäure, Fumarsäure, Itaconsäure, Crotonsäure, (Poly)ethylenglykol(meth)acrylat, (Poly)propylenglykol(meth)acrylat und (Poly)glycerin(meth)acrylat, ist.

7. Zusammensetzung für ein haftendes Hydrogel gemäss Anspruch 1, wobei (b) zumindest eine Art, ausgewählt aus der Gruppe bestehend aus tert-Butylacrylamidsulfonsäure (TBAS), tert-Butylacrylamidsulfonat, N,N-Dimethylaminoethylacrylamid (DMAEAA)-Hydrochlorid, N,N-Dimethylaminopropylacrylamid (DMAPAA)-Hydrochlorid, (Meth)acrylamid, N-Methyl(meth)acrylamid, N-Ethyl(meth)acrylamid, N-Propyl(meth)acrylamid, N,N-Dimethyl(meth)acrylamid, N,N-Diethyl(meth)acrylamid und Acryloylmorpholin, ist.

8. Zusammensetzung für ein haftendes Hydrogel gemäss Anspruch 1, wobei (d) zumindest eine Art, ausgewählt aus der Gruppe bestehend aus Methylenbis(meth)acrylamid, Ethylenbis(meth)acrylamid, (Poly)ethylenglycoldi-(meth)acrylat, (Poly)propylenglycoldi(meth)acrylat, 1,9-Nonandioldi(meth)acrylat, Glycerindi(meth)acrylat, Glycerintri(meth)acrylat, Tetraaryloxyethan und Diarylammoniumchlorid, ist.

9. Zusammensetzung für ein haftendes Hydrogel gemäss Anspruch 1, die ferner einen Elektrolyten umfasst.

10. Haftendes Hydrogel, hergestellt unter Verwendung einer Zusammensetzung für ein haftendes Hydrogel gemäss Anspruch 1, das zumindest eine durch Polymerisation eines polymeren Matrixbildners erhaltene Polymermatrix, Wasser und einen mehrwertigen Alkohol umfasst.

11. Gelschicht, hergestellt unter Verwendung eines haftenden Hydrogels gemäss Anspruch 10.

12. Elektrode, hergestellt unter Verwendung eines haftenden Hydrogels gemäss Anspruch 10.


**Revendications**

1. Composition pour un hydrogel adhésif, comprenant au moins : un agent de formation de matrice polymère ; de l'eau ; et un polyol ; l'agent de formation de matrice polymère comprenant au moins :

    (a) un acide (méth)acrylique, un dérivé d'acide (méth)acrylique ou un dérivé de vinyle ayant deux groupes

**EP 2 662 429 B1**

carboxyle et quatre ou cinq atomes de carbone ;
(b) un (méth) acrylamide ou un dérivé de (méth)acrylamide ;
(c) un N-vinyl-2-caprolactame et/ou un N-vinyl-2-valérolactame ; et
(d) un monomère réticulable,
dans laquelle la teneur en pourcentage du (b) dans la composition pour un hydrogel adhésif est de 2 à 20% en poids.

2. Composition pour un hydrogel adhésif de la revendication 1, dans laquelle la teneur en pourcentage du (a) est de 2 à 20% en poids, et la teneur en pourcentage du (c) est de 0,1 à 25% en poids.

3. Composition pour un hydrogel adhésif de la revendication 1, dans laquelle l'agent de formation de matrice polymère contient le (e) polyvinylpyrrolidone ou un dérivé de polyvinylpyrrolidone.

4. Composition pour un hydrogel adhésif de la revendication 3, dans laquelle la teneur en pourcentage du (e) est de 0,1 à 20% en poids.

5. Composition pour un hydrogel adhésif de la revendication 1, dans laquelle le polyol est au moins un type choisi dans le groupe constitué d'éthylène glycol, de propylène glycol, de butanediol, de glycérine, de pentaérythritol, de sorbitol, de polyéthylèneglycol, de polypropylèneglycol, de polyglycérol et de polyoxyéthylène polyglycéryl éther.

6. Composition pour un hydrogel adhésif de la revendication 1, dans laquelle le (a) est au moins un type choisi dans le groupe constitué d'acide (méth)acrylique, d'acide maléique, d'acide fumarique, d'acide itaconique, d'acide crotonique, de (méth)acrylate de (poly)éthylèneglycol, de (méth) acrylate de (poly)propylèneglycol et de (méth)acrylate de (poly)glycérine.

7. Composition pour un hydrogel adhésif de la revendication 1, dans laquelle le (b) est au moins un type choisi dans le groupe constitué d'acide tert-butylacrylamide sulfonique (TBAS), de sulfonate de tert-butylacrylamide, de chlorhydrate de N,N-diméthylaminoéthylacrylamide (DMAEAA), de chlorhydrate de N,N-diméthylaminopropylacrylamide (DMAPAA), de (méth)acrylamide, de N-méthyl(méth)acrylamide, de N-éthyl(méth)acrylamide, de N-propyl(méth)acrylamide, de N,N-diméthyl(méth)acrylamide, de N,N-diéthyl(méth)acrylamide et d'acryloylmorpholine.

8. Composition pour un hydrogel adhésif de la revendication 1, dans laquelle le (d) est au moins un type choisi dans le groupe constitué de méthylènebis(méth)acrylamide, d'éthylènebis(méth)acrylamide, de di(méth)acrylate de (poly)éthylèneglycol, de di(méth)acrylate de (poly)propylèneglycol, de di(méth)acrylate de 1,9-nonanediol, de di(méth)acrylate de glycérine, de tri(méth)acrylate de glycérine, de tétraaryloxyéthane et de chlorure de diarylammonium.

9. Composition pour un hydrogel adhésif de la revendication 1, comprenant en outre un électrolyte.

10. Hydrogel adhésif préparé en utilisant la composition pour l'hydrogel adhésif de la revendication 1, comprenant au moins une matrice polymère obtenue par polymérisation de l'agent de formation de matrice polymère, de l'eau et un polyol.

11. Feuille de gel préparée en utilisant l'hydrogel adhésif de la revendication 10.

12. Électrode préparée en utilisant l'hydrogel adhésif de la revendication 10.

**EP 2 662 429 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003096431 A
- JP 3437124 B